# EUROPEAN PATENT SPECIFICATION

(11) **EP 2 269 602 B1**
(45) Date of publication and mention of the grant of the patent: **19.11.2014**
(21) Application number: 10176412.4
(22) Date of filing: 29.09.1999
(51) Int. Cl.: A61K 31/435, A61K 31/47, A61K 31/535, A61K 31/57, A61P 27/02, A61P 27/16, A61K 45/06

(54) **Antibiotic compositions for treatment of the ear**
Antibiotische Zusammensetzungen zur Behandlung der Ohren
Compositions antibiotiques destinees au traitement des oreilles

(30) Priority: 30.09.1998 US 102504 P; 30.09.1998 US 102506 P
(43) Date of publication of application: 05.01.2011
(62) Divisional of application: 03020587.6
(73) Proprietor: Alcon Pharmaceuticals Ltd., 1701 Fribourg (CH)
(72) Inventor: Cagle, Gerald, Fort Worth, TX 76116 (US); Abshire, Robert L., Fort Worth, TX 76116 (US); Stroman, David W., Irving, TX 75063 (US); Yanni, John M., Burleson, TX 76028 (US)
(74) Representative: Best, Michael

(56) References cited:
- EP-A- 0 550 903
- WO-A-90/01933
- WO-A-96/39146
- WO-A-99/15172
- WO-A1-01/89485
- DE-A- 4 424 369
- US-A- 4 990 517
- US-A- 5 223 493
- US-A- 5 607 942
- KRASEMANN, C. (1) ET AL: "Efficacy of moxifloxacin against Staphylococcus aureus in respiratory tract and skin and skin structure infections." JOURNAL OF ANTIMICROBIAL CHEMOTHERAPY, (JULY, 1999) VOL. 44, NO. SUPPL. A, PP. 150. MEETING INFO.: 21ST INTERNATIONAL CONGRESS OF CHEMOTHERAPY BIRMINGHAM, ENGLAND, UK JULY 4-7, 1999, 7 July 1999 (1999-07-07), - 7 July 1999 (1999-07-07) XP000892776
- ELIES W.: "[Novel fluoroquinolones in the treatment of ENT infections]. NEUERE FLUORCHINOLONE BEI DER THERAPIE VON HNO-INFEKTIONEN." CHEMOTHERAPIE JOURNAL, (1998) 7/3 (93-97)., 1998, - 1998 XP000892813
- WOODCOCK J M ET AL: "In vitro activity of BAY 12-8039, a new fluoroquinolone." January 1997 (1997-01), ANTIMICROBIAL AGENTS AND CHEMOTHERAPY JAN 1997 LNKD- PUBMED:8980763, VOL. 41, NR. 1, PAGE(S) 101 - 106 , XP002608942 ISSN: 0066-4804 * See table 1 *
- STROMAN D W ET AL: "In Vitro and In Vivo Potency of Moxifloxacin and Moxifloxacin Ophthalmic Solution 0.5%, A New Topical Fluoroquinolone" SURVEY OF OPHTHALMOLOGY, SURVEY OF OPHTHALMOLOGY INC, XX, vol. 50, no. 6, 1 November 2005 (2005-11-01), pages S16-S31, XP025381754 ISSN: 0039-6257 [retrieved on 2005-11-01]
- ROBERTSON S M ET AL: "Ocular Pharmacokinetics of Moxifloxacin After Topical Treatment of Animals and Humans" SURVEY OF OPHTHALMOLOGY, SURVEY OF OPHTHALMOLOGY INC, XX, vol. 50, no. 6, 1 November 2005 (2005-11-01), pages S32-S45, XP025381755 ISSN: 0039-6257 [retrieved on 2005-11-01]
- DATABASE BIOSIS [Online] BIOSCIENCES INFORMATION SERVICE, PHILADELPHIA, PA, US 1989 KOUPPARI G ET AL: 'AEROBIC MICROORGANISMS ISOLATED FROM CHILDREN WITH OTORRHEA' Database accession no. PREV198988075067 & KOUPPARI G ET AL: "AEROBIC MICROORGANISMS ISOLATED FROM CHILDREN WITH OTORRHEA", DELTION ELLENIKES MIKROBIOLOGIKES ETAIREIAS, vol. 34, no. 3, 1989, pages 273-281, ISSN: 0438-9573
- OBI C L ET AL: "Bacterial agents causing chronic suppurative otitis media", EAST AFRICAN MEDICAL JOURNAL 1995 KE, vol. 72, no. 6, 1995, pages 370-372, ISSN: 0012-835X
- GARCIA-MARTOS P ET AL: "Nosocomial infection by Xanthomonas maltophilia: Antimicrobial resistance profile", REVISTA ESPANOLA DE QUIMIOTERAPIA 1995 ES, vol. 8, no. 2, 1995, pages 137-140, ISSN: 0214-3429
- ROTIMI V O ET AL: "The bacteriology of chronic suppurative otitis media.", EAST AFRICAN MEDICAL JOURNAL JUL 1992 LNKD- PUBMED:1396196, vol. 69, no. 7, July 1992 (1992-07), pages 394-397, ISSN: 0012-835X
- THORE M ET AL: "Efficacy of Metronidazole in experimental Bacteroides fragilis otitis media.", ACTA OTO-LARYNGOLOGICA 1985 JAN-FEB LNKD- PUBMED:3976396, vol. 99, no. 1-2, January 1985 (1985-01), pages 60-66, ISSN: 0001-6489
- FAIRBANKS D N: "Topical therapeutics for otitis media.", OTOLARYNGOLOGY--HEAD AND NECK SURGERY : OFFICIAL JOURNAL OF AMERICAN ACADEMY OF OTOLARYNGOLOGY-HEAD AND NECK SURGERY 1981 MAY-JUN LNKD- PUBMED:6791095, vol. 89, no. 3 Pt 1, May 1981 (1981-05), pages 381-385, ISSN: 0194-5998
- COOPER M A ET AL: "Ciprofloxacin resistance developing during treatment of malignant otitis externa.", THE JOURNAL OF ANTIMICROBIAL CHEMOTHERAPY JUL 1993 LNKD- PUBMED:8226407, vol. 32, no. 1, July 1993 (1993-07), pages 163-164, ISSN: 0305-7453

## Description

### Background of the Invention

The present invention is directed to the provision of topical antibiotic pharmaceutical compositions for the treatment of otic infections associated with P. aeruginosa, H. influenzae β-lactamase positive, or H. influenzae β-lactamase negative. Such compositions are useful in methods of treating otic infections by applying those compositions to the affected tissues. The compositions and methods of the invention are based on the use of Moxifloxacin. The compositions of the present invention may also contain one or more anti-inflammatory agents.

The use of quinolone antibiotics to treat infections represents the current state of the art in the field of ophthalmic pharmaceutical compositions and methods of treatment. For example, a topical ophthalmic composition containing the quinolone ciprofloxacin is marketed by Alcon Laboratories, Inc. under the name of CILOXAN^{™} (Ciprofloxacin 0.3%) Ophthalmic Solution. The following quinolones have also been utilized in ophthalmic antibiotic compositions:

| **Quinolone** | **Product** | **Manufacturer** |
|---|---|---|
| Ofloxacin | OCUFLOX™ | Allergan |
| Norfloxacin | CHIBROXIN™ | Merck |
| Lomefloxacin | LOMEFLOX™ | Senju |

The foregoing quinolone antibiotic compositions are generally effective in treating ophthalmic infections, and have distinct advantages over prior ophthalmic antibiotic compositions, particularly those having relatively limited spectrums of antimicrobial activity, such as: neomycin, polymyxin B, gentamicin and tobramycin,
which are primarily useful against gram negative pathogens; and bacitracin, gramicidin, and erythromycin, which are primarily active against gram positive pathogens. However, despite the general efficacy of the ophthalmic quinolone therapies currently available, there is a need for improved compositions and methods of treatment based on the use of antibiotics that are more effective than existing antibiotics against key ophthalmic pathogens, and less prone to the development of resistance by those pathogens.

WO 96/39146 discloses otic antibacterial compositions comprising ciprofloxacin.

There is an even greater need for effective topical compositions and methods for treating otic infections, particularly bacterial infections. The use of oral antibiotics to treat otic infections in children has limited efficacy, and creates a serious risk of pathogen resistance to the orally administered antibiotics.

Otic infections are frequently accompanied by inflammation of the infected otic tissues and perhaps even surrounding tissues. Similarly, otic surgical procedures that create a risk of microbial infections frequently also cause inflammation of the affected tissues. Thus, there is also a need for otic pharmaceutical compositions that combine the anti-infective activity of one or more antibiotics with the anti-inflammatory activity of one or more steroid or non-steroid agents in a single composition.

### Summary of the invention

The invention is based on the use of a potent antibiotic to treat otic infections associated with P. aeruginosa, H. influenzae / β-lactamase positive, or H. influenzae / β-lactamase negative, as well as the prophylactic use of these antibiotics following surgery or other trauma to otic tissues. The compositions of the present invention may also be administered to the affected tissues during otic surgical procedures to prevent or alleviate post-surgical infection.

The compositions preferably also contain one or more anti-inflammatory agents to treat inflammation associated with infections of otic tissues. The anti-inflammatory component of the compositions is also useful in treating inflammation associated with physical trauma to otic tissues, including inflammation resulting from surgical procedures. The compositions of the present invention are therefore particularly useful in treating inflammation associated with trauma to otic tissues wherein there is either an infection or a risk of an infection resulting from the trauma.

Examples of otic conditions that may be treated with the compostions of the present invention include otitis externa and otitis media. With respect to the treatment of otitis media, the compositions of the present invention are primarily useful in cases where the tympanic membrane has ruptured or tympanostomy tubes have been implanted. The compositions may also be used to treat infections associated with otic surgical procedures, such as tympanostomy, or to prevent such infections.

The compositions of the present invention are specially formulated for topical application to otic tissues. The compositions are preferably sterile, and have physical properties (e.g., osmolality and pH) that are specially suited for application to otic tissues, including tissues that have been compromised as the result of preexisting disease, trauma, surgery or other physical conditions.

### Detailed Description of the Invention

The antibiotic used in the compositions and methods of the present invention. is Moxifloxacin or a pharmaceutically useful hydrate or salt thereof. Moxifloxacin has the following structure:

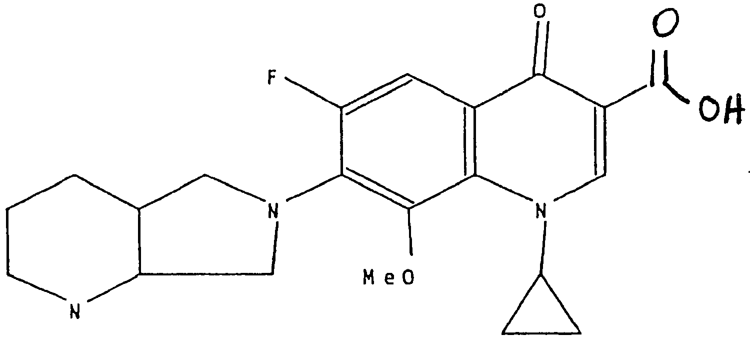

The concentrations of the antibiotic in the composition of the present invention will vary depending on the intended use of the compositions (e.g., treatment of existing infections or prevention of post-surgical infections), and the relative antimicrobial activity of the specific antibiotic selected. The antimicrobial activity of antibiotics is generally expressed as the minimum concentration required to inhibit the growth of a specified pathogen. This concentration is also referred to as the "minimum inhibitory concentration" or "MIC". The term "MIC90" refers to the minimum concentration of antibiotic required to inhibit the growth of ninety percent (90%) of the strains of a species. The concentration of an antibiotic required to totally kill a specified bacteria is referred to as the "minimum bactericidal concentration" or "MBC". The minimum inhibitory concentration of Moxifloxacin for several bacteria commonly associated with otic infections are provided in the following table:

| Microorganism | MIC ₉₀ |
|---|---|
| S. aureus/methicillin sensitive | 0.13 |
| S. aureus/methicillin resistant | 4.0 |
| S. aureus/quinolone resistant | 4.0 |
| S. epidermidis/methicillin sensitive | 0.25 |
| S. epidermidis/methicillin resistant | 4.0 |
| S. pneumoniae/penicillin sensitive | 0.25 |
| S. pneumoniae/penicillin resistant | 0.25 |
| P. aeruginosa | 8.0 |
| H. influenzae/β-lactamase positive | 0.06 |
| H influenzae/βlactamase negative | 0.06 |

All of the foregoing concentrations are expressed as micrograms per milliliter ("mcg/ml").

The appropriate concentration for otic compositions will generally be an amount of Moxifloxacin or a pharmaceutical useful hydrate or salt thereof sufficient to provide a concentration in the infected tissues equal to or greater than the MIC90 level for the selected antibiotic(s), relative to gram-negative and gram-positive organisms commonly associated with otic infections. Such amounts are referred to herein as "an antimicrobial effective amount". The compositions of the present invention will typically contain Moxifloxacin or a pharmaceutical useful hydrate or salt thereof in a concentration of from about 0.1 to about 1.0 percent by weight ("wt.%") of the compositions.

The compositions of the present invention may also contain one or more anti-inflammatory agents. The anti-inflammatory agents utilized in the present invention are broadly classified as steroidal or non-steroidal. The preferred steroidal anti-inflammatory agents are glucocorticoids.

The preferred glucocorticoids for otic use include dexamethasone, loteprednol, rimexolone, prednisolone, fluorometholone, and hydrocortisone. The preferred glucocorticoids for nasal use include mometasone, fluticasone, beclomethasone, flunisolide, triamcinolone and budesonide.

The dexamethasone derivatives described in U.S. Patent No. 5,223,493 (Boltralik) are also preferred steroidal anti-inflammatory agents. The following compounds are especially preferred:

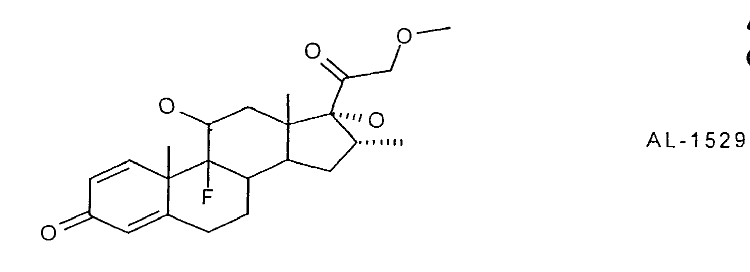

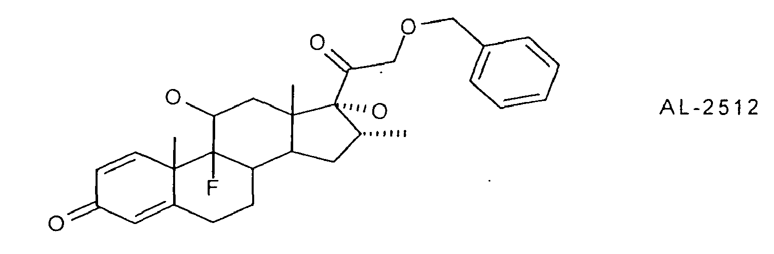

These compounds are referred to herein as "21-ether derivatives of dexamethasone". The 21-benzyl ether derivative (i.e., compound AL-2512) is particularly preferred.

The preferred non-steroidal anti-inflammatory agents are: prostaglandin H synthetase inhibitors (Cox I or Cox II), also referred to as cyclooxygenase type I and type II inhibitors, such as diclofenac, flurbiprofen, ketorolac, suprofen, nepafenac, amfenac, indomethacin, naproxen, ibuprofen, bromfenac, ketoprofen, meclofenamate, piroxicam, sulindac, mefanamic acid, diflusinal, oxaprozin, tolmetin, fenoprofen, benoxaprofen, nabumetome, etodolac, phenylbutazone, aspirin, oxyphenbutazone, NCX-4016, HCT-1026, NCX-284, NCX-456, tenoxicam and carprofen; cyclooxygenase type II selective inhibitors, such as NS-398, vioxx, celecoxib, P54, etodolac, L-804600 and S-33516; PAF antagonists, such as SR-27417, A-137491, ABT-299, apafant, bepafant, minopafant, E-6123, BN-50727, nupafant and modipafant; PDE IV inhibitors, such as ariflo, torbafylline, rolipram, filaminast, piclamilast, cipamfylline, CG-1088, V-11294A, CT-2820, PD-168787, CP-293121, DWP-205297, CP-220629, SH-636, BAY-19-8004, and roflumilast; inhibitors of cytokine production, such as inhibitors of the NFkB transcription factor; or other anti-inflammatory agents known to those skilled in the art.

The concentrations of the anti-inflammatory agents contained in the compositions of the present invention will vary based on the agent or agents selected and the type of inflammation being treated. The concentrations will be sufficient to reduce inflammation in the targeted otic tissues following topical application of the compositions to those tissues. Such an amount is referred to herein as "an anti-inflammatory effective amount". The compositions of the present invention will typically contain one or more anti-inflammatory agents in an amount of from about 0.01 to about 1.0 wt.%.

The compositions are typically administered to the affected otic tissues by topically applying one to four drops of a sterile solution or suspension, or a comparable amount of an ointment, gel or other solid or semisolid composition, one to four times per day. However, the compositions may also be formulated as irrigating solutions that are applied to the affected otic tissues during surgical procedures.

The otic compositions of the present invention will contain one or more compounds of formula (I) and preferably one or more anti-inflammatory agents, in pharmaceutically acceptable vehicles. The compositions will typically have a pH in the range of 4.5 to 8.0. The ophthalmic compositions must also be formulated to have osmotic values that are compatible with the aqueous humor of the eye and ophthalmic tissues. Such osmotic values will generally be in the range of from about 200 to about 400 milliosmoles per kilogram of water ("mOsm/kg"), but will preferably be about 300 mOsm/kg.

Otic pharmaceutical products are typically packaged in multidose form. Preservatives are thus required to prevent microbial contamination during use. Suitable preservatives include: polyquaternium-1, benzalkonium chloride, thimerosal, chlorobutanol, methyl paraben, propyl paraben, phenylethyl alcohol, edetate disodium, sorbic acid, or other agents known to those skilled in the art. The use of polyquaternium-1 as the antimicrobial preservative is preferred. Typically such preservatives are employed at a level of from 0.001% to 1.0% by weight.

The solubility of the components of the present compositions may be enhanced by a surfactant or other appropriate co-solvent in the composition. Such co-solvents include polysorbate 20, 60, and 80, polyoxyethylene/polyoxypropylene surfactants (e.g., Pluronic F-68, F-84 and P-103), cyclodextrin, or other agents known to those skilled in the art. Typically such co-solvents are employed at a level of from 0.01 % to 2% by weight.

The use of viscosity enhancing agents to provide the compositions of the invention with viscosities greater than the viscosity of simple aqueous solutions may be desirable to increase the retention time in the ear. Such viscosity building agents include, for example, polyvinyl alcohol, polyvinyl pyrrolidone, methyl cellulose, hydroxy propyl methylcellulose, hydroxyethyl cellulose, carboxymethyl cellulose, hydroxy propyl cellulose or other agents know to those skilled in the art. Such agents are typically employed at a level of from 0.01 % to 2% by weight.

The following examples are provided to further illustrate the otic compositions of the present invention.

### Example 1

### Otic Solution

| **Ingredient** | **Amount (wt. %)** |
|---|---|
| Moxifloxacin | 0.35 |
| Sodium Acetate | 0.03 |
| Acetic Acid | 0.04 |
| Mannitol | 4.60 |
| EDTA | 0.05 |
| Benzalkonium Chloride | 0.006 |
| Water | q.s. 100 |

### Example 2

### Otic Suspension

| **Ingredient** | **Amount (wt. %)** |
|---|---|
| Moxifloxacin 0.3 | |
| Dexamethasone, Micronized USP | 0.10 |
| Benzalkonium Chloride | 0.01 |
| Edetate Disodium, USP | 0.01 |
| Sodium Chloride, USP | 0.3 |
| Sodium Sulfate, USP | 1.2 |
| Tyloxapol, USP | 0.05 |
| Hydroxyethylcellulose | 0.25 |
| Sulfuric Acid and/or | |
| Sodium Hydroxide, NF | q.s. for pH adjustment to 5.5 |
| Purified Water, USP | q.s. to 100 |

The invention has been described herein by reference to certain preferred embodiments. However, as obvious variations thereon will become apparent to those skilled in the art, the invention is not to be considered as limited thereto.

## Claims

1. Moxifloxacin or a pharmaceutically useful hydrate or salt thereof for use in topical treating or preventing otic infections associated with *P. aeruginosa, H. influenzae* / *β-lactamase positive,* or *H. influenzae* / *β-lactamase negative.*

2. Moxifloxacin or a pharmaceutically useful hydrate or salt thereof for a use according to claim 1, wherein the otic infection is associated with *P. aeruginosa.*

3. Moxifloxacin or a pharmaceutically useful hydrate or salt thereof for a use according to claim 1, wherein the otic infection is associated with *H. influenzae* / *β-lactamase positive,* or *H. influenzae* / *β-lactamase negative.*

4. Topical otic composition comprising an antimicrobial effective amount of Moxifloxacin or a pharmaceutically useful hydrate or salt thereof and a pharmaceutically acceptable vehicle therefore for use in treating or preventing otic infections associated with *P. aeruginosa, H. influenzae* / *β-*/*actamase positive,* or *H. influenzae* / *β-lactamase negative.*

5. Composition for use according to claim 4 comprising Moxifloxacin or a pharmaceutically useful hydrate or salt thereof in a concentration of 0.1 to 1.0 weight-%.

6. Composition for use according to any one of claims 4 or 5, wherein the treatment or prevention of otic infections associated with *P. aeruginosa, H. influenzae* / *β-lactamase positive,* or *H. influenzae* / *β-*/*actamase negative* comprises topically applying a therapeutically effective amount of the composition to the affected otic tissue.

7. Composition for use according to any one of claims 4 to 6, wherein the composition further comprises an anti-inflammatory effective amount of a steroidal or non-steroidal anti-inflammatory agent.

8. Composition for use according to claim 7, wherein the anti-inflammatory agent comprises a glucocorticoid.

9. Composition for use according to claim 8, wherein the glucocorticoid is selected from the group consisting of dexamethasone, rimexolone, prednisolone, fluorometholone, hydrocortisone, mometasone, fluticasone, beclomethasone, flunisolide, triamcinolone and budesonide.

10. Composition for use according to claim 7, wherein the anti-inflammatory agent comprises a non-steroidal agent selected from the group consisting of prostaglandin H synthetase inhibitors, PAF antagonists, and PDE IV inhibitors.

11. Composition for use according to claim 7, wherein the anti-inflammatory agent comprises dexamethasone.

## Patentansprüche

1. Moxifloxacin oder ein pharmazeutisch verwendbares Hydrat oder Salz davon zur Verwendung bei der topischen Behandlung oder Vorbeugung von Infektionen des Ohrs, die mit *P. aeruginosa, H. influenzae*/*β-Lactamase positiv* oder *H. influenzae*/*β-Lactamase-negativ* in Verbindung stehen.

2. Moxifloxacin oder ein pharmazeutisch verwendbares Hydrat oder Salz davon zur Verwendung nach Anspruch 1, wobei die Infektion des Ohrs mit *P. aeruginosa* in Verbindung steht.

3. Moxifloxacin oder ein pharmazeutisch verwendbares Hydrat oder Salz davon zur Verwendung nach Anspruch 1, wobei die Infektion des Ohrs mit *H. influenzae*/*β-Lactamase-positiv* oder *H. influenzae*/*β-Lactamase-negativ* in Verbindung steht.

4. Topische Zusammensetzung für das Ohr, umfassend eine antimikrobielle wirksame Menge Moxifloxacin oder ein pharmazeutisch verwendbares Hydrat oder Salz davon und ein pharmazeutisch akzeptables Vehikel dafür zur Verwendung bei der Behandlung oder Vorbeugung von Infektionen des Ohrs, die mit *P. aeruginosa, H. influenzae*/*β-Lactamase-positiv* oder *H. influenzae*/*β-Lactamase-negativ* in Verbindung stehen.

5. Zusammensetzung zur Verwendung nach Anspruch 4, umfassend Moxifloxacin oder ein pharmazeutisch verwendbares Hydrat oder Salz davon in einer Konzentration von 0,1 bis 1,0 Gew.-%.

6. Zusammensetzung zur Verwendung nach einem der Ansprüche 4 oder 5, wobei die Behandlung oder Vorbeugung von Infektionen des Ohrs, die mit *P. aeruginosa, H. influenzae*/*β-Lactamase positiv* oder *H. influenzae*/*β-Lactamase-negativ* in Verbindung stehen, das topische Auftragen einer therapeutisch wirksamen Menge der Zusammensetzung auf das betroffene Ohrgewebe umfasst.

7. Zusammensetzung zur Verwendung nach einem der Ansprüche 4 bis 6, wobei die Zusammensetzung ferner eine entzündungshemmende wirksame Menge eines steroidalen oder nicht steroidalen Entzündungshemmers umfasst.

8. Zusammensetzung zur Verwendung nach Anspruch 7, wobei der Entzündungshemmer ein Glucocorticoid umfasst.

9. Zusammensetzung zur Verwendung nach Anspruch 8, wobei das Glucocorticoid aus der Gruppe ausgewählt ist, bestehend aus Dexamethason, Rimexolon, Prednisolon, Fluorometholon, Hydrocortison, Mometason, Fluticason, Beclomethason, Flunisolid, Triamcinolon und Budesonid.

10. Zusammensetzung zur Verwendung nach Anspruch 7, wobei der Entzündungshemmer ein nicht steroidales Mittel, ausgewählt aus der Gruppe, bestehend aus Prostaglandin-H-Synthetaseinhibitoren, PAF-Antagonisten und PDE-IV-Inhibitoren, umfasst.

11. Zusammensetzung zur Verwendung nach Anspruch 7, wobei der Entzündungshemmer Dexamethason umfasst.

## Revendications

1. Moxifloxacine ou un hydrate ou un sel de Moxifloxacine, ledit hydrate et ledit sel présentant une utilité pharmaceutique, pour utilisation dans un traitement topique ou dans la prévention des infections otiques associées à *P. aeruginosa, H. influenzae* / *β-lactamase positive ou H. influenzae* / *β-lactamase négative.*

2. Moxifloxacine ou un hydrate ou un sel de Moxifloxacine, ledit hydrate et ledit sel présentant une utilité pharmaceutique, pour une utilisation selon la revendication 1, dans laquelle l'infection otique est associée à *P. aeruginosa.*

3. Moxifloxacine ou un hydrate ou un sel de Moxifloxacine, ledit hydrate et ledit sel présentant une utilité pharmaceutique, pour une utilisation selon la revendication 1, dans laquelle l'infection otique est associée à *H. influenzae* / *β-lactamase positive, ou H. influenzae* / *β-lactamase négative.*

4. Composition otique topique comprenant une quantité antimicrobienne effective de Moxifloxacine ou un hydrate ou un sel de Moxifloxacine, ledit hydrate et ledit sel présentant une utilité pharmaceutique, et un véhicule pharmaceutique acceptable par conséquent pour utilisation dans un traitement ou une prévention d'infections otiques associées à *P. aeruginosa, H. influenzae* / *β-lactamase positive, ou H. influenzae* / *β-lactamase négative.*

5. Composition pour utilisation selon la revendication 4 comprenant de la Moxifloxacine ou un hydrate ou un sel de Moxifloxacine, ledit hydrate et ledit sel présentant une utilité pharmaceutique, dans une concentration allant de 0,1 à 1,0 en pourcentage massique.

6. Composition pour utilisation selon l'une quelconque des revendications 4 ou 5, dans laquelle le traitement ou la prévention des infections otiques associées à *P. aeruginosa, H. influenzae* / *β-lactamase positive, ou H. influenzae* / *β-lactamose négative* comprend une application topique de ladite composition au tissu otique affecté dans une quantité thérapeutiquement effective.

7. Composition pour utilisation selon l'une quelconque des revendications 4 à 6, dans laquelle la composition comprend en outre un agent anti-inflammatoire stéroïde ou non-stéroïde dans une quantité effective sur le plan anti-inflammatoire.

8. Composition pour utilisation selon la revendication 7, dans laquelle l'agent anti-inflammatoire comprend un glucocorticoïde.

9. Composition pour utilisation selon la revendication 8, dans laquelle le glucocorticoïde est sélectionné à partir du groupe constitué de dexaméthasone, de riméxolone, de prednisolone, de fluorométholone, d'hydrocortisone, de mométasone, de fluticasone, de béclométhasone, de flunisolide, de triamcinolone et de budésonide.

10. Composition pour utilisation selon la revendication 7, dans laquelle l'agent anti-inflammatoire comprend un agent non-stéroïde sélectionné à partir du groupe constitué d'inhibiteurs de prostaglandine H synthétase, d'antagonistes PAF, et d'inhibiteurs PDE 4.

11. Composition pour utilisation selon la revendication 7, dans laquelle l'agent anti-inflammatoire comprend de la dexaméthasone.
